# EUROPEAN PATENT APPLICATION

(11) **EP 3 991 636 A1**
(43) Date of publication of application: **04.05.2022**
(21) Application number: 19935118.0
(22) Date of filing: 28.06.2019
(51) Int. Cl.: A61B 5/00

(54) **MONITORING APPARATUS AND METHOD FOR OPERATING SAME, MONITOR AND COMPUTER STORAGE MEDIUM**

(71) Applicant: Shenzhen Mindray Bio-Medical Electronics Co., Ltd., Shenzhen, Guangdong 518057 (CN)
(72) Inventor: SUN, Zehui, Shenzhen, Guangdong 518057 (CN); YE, Wenyu, Shenzhen, Guangdong 518057 (CN); GUAN, Zehong, Shenzhen, Guangdong 518057 (CN); LIU, Sanchao, Shenzhen, Guangdong 518057 (CN); DAI, Jian, Shenzhen, Guangdong 518057 (CN); HE, Xianliang, Shenzhen, Guangdong 518057 (CN)
(74) Representative: KIPA AB
(86) International application number: PCT/CN2019/093927
(87) International publication number: WO 2020/258337

(57) **Abstract**

Disclosed are a monitoring apparatus and a method for operating same, a monitor and a computer storage medium. The method comprises: during the process of monitoring a monitored object, acquiring at least one type of monitoring signal of the monitored object in real time by means of a sensor accessory (111) connected to the monitored object; acquiring at least one type of monitoring parameter (101) of the monitored object according to the monitoring signal, and analyzing the at least one type of monitoring parameter according to a first data analysis function; and when the at least one type of monitoring parameter satisfies a preset condition, executing a data analysis function changing step (102), the data analysis function changing step comprising at least one of the following steps: activating a second data analysis function for at least one type of physiological parameter of the monitored object, and deactivating the second data analysis function for at least one type of physiological parameter of the monitored object, wherein the monitoring parameter is at least used for representing one of the following features of the monitored object: a physiological state, a motion state and a posture state. Thus, a monitoring function for a physiological parameter of a monitored object can be activated or deactivated according to the current monitoring parameter of the monitored object, such that a monitored physiological parameter can be adaptively adjusted, thereby improving the monitoring quality.

## Description

### TECHNICAL FIELD

The disclosure relates to the technical field of monitoring a monitored object, and more particularly to a monitoring apparatus, a method for operating a monitoring apparatus, a monitor and a computer storage medium.

### BACKGROUND

In related technologies, the monitoring mode of the monitor is passive. Specifically, the working mode of the monitor is to monitor parameters according to the user's settings. But the monitor cannot adjust the monitored parameters intelligently according to the changes of the state of the monitored object. In this way, it may lead to untimely capture of the changes in the states of the monitored object, reduce the monitoring quality, and further prolong the hospitalization time of the monitored object and increase the medical burden.

### SUMMARY

The embodiments of this disclosure have provided a monitoring apparatus, a method for operating a monitoring apparatus, a monitor and a computer storage medium, capable of adaptively and timely reminding the user to adjust and/or adaptively and timely adjusting monitored physiological parameters to improve the monitoring quality.

The embodiments of this disclosure have provided a monitoring apparatus, which includes a processor and a parameter measurement circuit; wherein
the parameter measurement circuit is configured to acquire a monitored signal of a monitored object in real time through a sensor accessory connected with the monitored object in a monitoring process of the monitored object; and
the processor is configured to acquire a monitored parameter of the monitored object according to the monitored signal, and analyze the monitored parameter according to a first data analysis function; wherein the monitored parameter includes at least a first physiological parameter for characterizing a physiological state of the monitored object;
wherein when the monitored parameter satisfies a preset requirement, the processor is configured to perform a data analysis function change step, wherein the data analysis function change step includes at least one of the following steps:
   turning on a second data analysis function for the first physiological parameter; and
   turning off the second data analysis function for the first physiological parameter.

The embodiments of this disclosure have provided a method for operating a monitoring apparatus, which includes:
acquiring at least one monitored signal of a monitored object in real time in a monitoring process of the monitored object; and
acquiring a monitored parameter of the monitored object according to the monitored signal, and analyzing the monitored parameter according to a first data analysis function; wherein the monitored parameter includes at least a first physiological parameter for characterizing a physiological state of the monitored object;
wherein when the monitored parameter satisfies a preset requirement, performing a data analysis function change step, wherein the data analysis function change step includes at least one of the following steps:
   turning on a second data analysis function for the first physiological parameter; and
   turning off the second data analysis function for the first physiological parameter.
   The embodiments of this disclosure have provided a further monitoring apparatus, which includes a processor and a parameter measurement circuit;
   wherein the parameter measurement circuit is configured to acquire at least one monitored signal of a monitored object in real time through a sensor accessory connected with the monitored object in a monitoring process of the monitored object; and
   the processor is configured to acquire at least one monitored parameter of the monitored object according to the monitored signal, and analyze the at least one monitored parameter according to a first data analysis function;
wherein when the at least one monitored parameter satisfies a preset requirement, the processor is configured to perform a data analysis function change step, wherein the data analysis function change step includes at least one of the following steps:
   turning on a second data analysis function for at least one physiological parameter; and
   turning off the second data analysis function for at least one physiological parameter;
   wherein the monitored parameter is at least operable to characterize at least one of following characteristics of the monitored object: a physiological state, a motion state and a posture state.

The embodiments of this disclosure have provided a monitor, which includes any one of the above monitoring apparatuses.

The embodiments of this disclosure have provided a further method for operating a monitoring apparatus, which includes:
acquiring at least one monitored signal of a monitored object in real time through a sensor accessory connected with the monitored object in a monitoring process of the monitored object; and
acquiring at least one monitored parameter of the monitored object according to the monitored signal, and analyze the at least one monitored parameter according to a first data analysis function;
wherein when the at least one monitored parameter satisfies a preset requirement, performing a data analysis function change step, wherein the data analysis function change step includes at least one of the following steps:
   turning on a second data analysis function for at least one physiological parameter of the monitored object; and
   turning off the second data analysis function for at least one physiological parameter of the monitored object;
   wherein the monitored parameter is at least operable to characterize at least one of following characteristics of the monitored object: a physiological state, a motion state and a posture state.

The embodiments of this disclosure have provided a computer storage medium, which includes a computer program that can be executed by a processor to implement any one method for operating a monitoring apparatus described above.

In the technical schemes provided by the embodiment of this disclosure, in the monitoring process of the monitored object, at least one monitored signal of the monitored object is obtained in real time through the sensor accessory connected with the monitored object, at least one monitored parameter of the monitored object is obtained according to the monitored signal, the at least one monitored parameter is analyzed according to a first data analysis function. When the at least one monitored parameter satisfied a preset requirement, a data analysis function change step is performed. The data analysis function change step includes at least one of the following steps: turning on a second data analysis function for at least one physiological parameter of the monitored object; and turning off the second data analysis function for at least one physiological parameter of the monitored object. Wherein the monitored parameters are operable to characterize at least one of the following characteristics of the monitored object: a physiological state, a motion state and a posture state. In such a way, the embodiment of this disclosure can turn on or off the data analysis function for the physiological parameter of the monitored object according to the current monitored parameter of the monitored object. That is, the monitored physiological parameter can be adjusted adaptively to improve the monitoring quality.

### BRIEF DESCRIPTION OF THE DRAWINGS

FIG. 1 is a flowchart of a method for operating a monitoring apparatus according to an embodiment of this disclosure.
FIG. 2 is a system framework diagram of a multi-parameter monitor or module component according to an embodiment of this disclosure.
FIG. 3 is a frame diagram of a monitoring device networking system according to an embodiment of this disclosure.
Fig. 4 is a structural diagram of a monitoring apparatus according to an embodiment of this disclosure.

### DESCRIPTION OF THE PREFERRED EMBODIMENTS

In order to understand the characteristics and technical content of the embodiment of this disclosure more detail, the implementation of the embodiment of this disclosure is described in detail below in combination with the attached drawings. The attached drawings are for reference and explanation only and are not operable to limit the embodiment of this disclosure.

The terms "first", "second", etc. in the specification and the claims herein as well as the above accompanying drawings are operable to distinguish different objects, rather than to describe a specific order. In addition, the terms "comprising", "having", and any variations thereof are intended to cover non-exclusive inclusion. For example, a process, a method, a system, a product, or a device that includes a series of steps or units is not limited to the listed steps or units, but optionally further includes unlisted steps or units, or optionally further includes other steps or units inherent in these processes, methods, or devices.

In related technologies, the monitoring scheme adopted by the monitor is a passive monitoring scheme. For example, after receiving a cardiopath, the department of cardiac intensive care unit (CCU) often only monitors ECG parameters, but does not pay attention to blood oxygen, blood pressure and other parameters. However, in the diagnosis and treatment process of the monitored object, the state change of the monitored object may affect the indexes of blood oxygen and blood pressure. When the illness symptoms of the monitored object occur and the medical staff finds that it is not enough to monitor the ECG parameters only according to the change of the illness state of the monitored object or through reviewing the history, he/she should set the relative parameter measurement manually. In this way, the tracking of the illness state of the monitored object is often not timely, and even the illness state of the monitored object cannot be captured, thus delaying the treatment of the illness state. Passive monitoring does not capture the changes of patients' illness state in time, which will inevitably lead to the extension of patients' hospitalization time, increase the medical burden and reduce the monitoring quality.

Aiming at the above technical problems, the embodiment of this disclosure provides a method for operating a monitoring apparatus, which can be applied to the monitoring apparatus, such as the monitor to monitor the parameters of the monitored object.

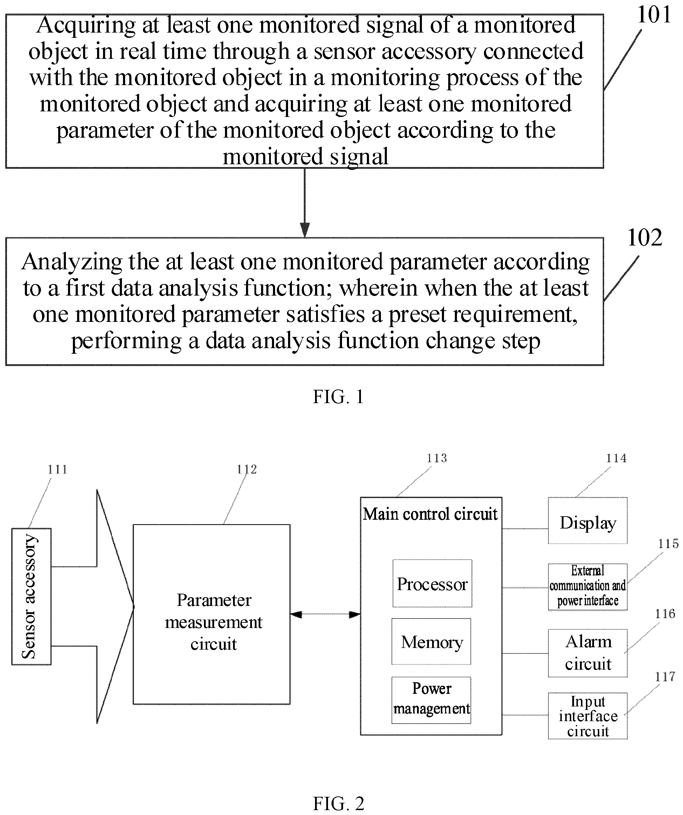

FIG. 1 is a flowchart of a method for operating a monitoring apparatus according to an embodiment of this disclosure. As shown in FIG. 1, the method can include the following steps.

In step 101, at least one monitored signal of a monitored object is acquired in real time through a sensor accessory connected with the monitored object in a monitoring process of the monitored object, and at least one monitored parameter of the monitored object is obtained according to the monitored signal.

Wherein the monitored parameter is at least operable to characterize at least one of the following characteristics of the monitored object: a physiological state, a motion state and a posture state.

Several implementation manners of this step are described below by way of example.

### Implementation manner one

Regarding to the implementation manner of acquiring at least one monitored parameter of the monitored object, in one example, the above at least one monitored parameter may include a first physiological parameter of the monitored object. The first physiological parameter can be operable to characterize a physiological state of the monitored object. Alternatively, the first physiological parameter of the monitored object is determined according to the actual monitoring requirement of the monitored object. For example, the first physiological parameter of the monitored object may be an ECG monitored parameter or other physiological monitored parameters.

In practical application, a monitoring apparatus, such as a monitor can be operable to acquire the first physiological parameter of the monitored object. After acquiring the first physiological parameter of the monitored object, the first physiological parameter of the monitored object can also be stored.

### Implementation manner two

Regarding to the implementation manner of acquiring at least one monitored parameter of the monitored object, in another example, a motion state data and/or a posture state data of the monitored object can be acquired. Herein, the motion state data of the monitored object can represent the acceleration, speed and other information of the monitored object. The posture state data of the monitored object can represent the posture information of the monitored object. It can be understood that the motion state and/or posture information of the monitored object can be determined according to the motion state data and/or posture state data of the monitored object. For example, it can be determined whether the monitored object is exercising violently.

In the embodiment of this disclosure, at least one motion signal of the monitored object can be monitored, and the motion state data and/or posture state data of the monitored object can be obtained according to the at least one motion signal of the monitored object. In practical application, the motion signal (such as acceleration data) of the monitored object can be obtained by a sensor, such as an accelerometer and a gyroscope. Then the motion state data and/or posture state data of the monitored object can be calculated based on the motion signal of the monitored object. After acquiring the motion state data and/or posture state data of the monitored object, the motion state data of the monitored object can also be stored.

In step 102, the at least one monitored parameter is analyzed according to a first data analysis function; wherein when the at least one monitored parameter satisfies a preset requirement, a data analysis function change step is performed.

Wherein the data analysis function change step includes at least one of the following steps: turning on a second data analysis function for at least one physiological parameter of the monitored object for indicating a real-time change of the at least one physiological parameter; and turning off the second data analysis function for at least one physiological parameter of the monitored object.

Understandably, when the at least one monitored parameter satisfies a preset requirement, a second data analysis function for at least one physiological parameter of the monitored object is turned on or off. Herein, "turn on" or "turn off" includes directly and intelligently turning on or off the second data analysis function for at least one physiological parameter of the monitored object. Moreover, "turn on" or "turn off' can also include sending a first indication information to indicate the user to turn on or off the second data analysis function for the at least one physiological parameter, and then turning on or off the second data analysis function for the at least one physiological parameter after receiving a confirmation instruction. The form of the first indication information is not limited, and it can be realized in the form of a preset sound, light, text, graphics, etc., as long as it can remind the user to turn on or off the second data analysis function for the at least one physiological parameter.

Further, when the second data analysis function for the at least one physiological parameter of the monitored object is turned on or off, a second indication information is sent out. The second indication information includes at least one of the followings: the preset requirement, the monitored parameter, the physiological parameter, the data analysis function which is turned on or off. In such a way, from the second indication information, the user can know the second data analysis function which is turned on, the physiological parameter corresponding to the second data analysis function which is currently turned on, the reason for turning on the second data analysis function and other information, so as to monitor the patient in a more timely manner. Understandably, the form of the second indication information is not limited, and it can also be realized in the form of a preset sound, light, text, graphics, etc., as long as it can remind the user to turn on or off the data analysis function for the at least one physiological parameter.

Among others, the first data analysis function and the second data analysis function include at least one of the following functions: a data measurement function for acquiring a physiological characteristic signal in real time to acquire at least one physiological parameter according to the physiological characteristic signal; a data processing function for processing at least one physiological parameter according to a preset rule; and a monitoring function for generating an alarm information when at least one physiological parameter satisfies a preset alarm requirement.

Regarding to the implementation manner of this step, for example, the at least one monitored parameter includes a first physiological parameter of the monitored object, and the at least one physiological parameter includes a first physiological parameter. It can be understood that, at this time, the monitored signal and the physiological characteristic signal can be generated from the same signal source. For example, both the monitored signal and the physiological characteristic signal are ECG signals. Thus, when at least one preset physiological signal characteristic is determined to change according to the change of the first physiological parameter, a second data analysis function for the first physiological parameter can be turned on or off.

Regarding to the implementation manner of turning on the second data analysis function for the first physiological parameter, the following takes ST wave in the ECG signal as an example. In the ECG monitoring process, when the patient has myocardial ischemia or injury, the ST wave in the ECG signal will deviate from a zero-point potential difference line. The risk of myocardial ischemia or infarction of the patient can be identified by the deviation value of the ST wave segment. The monitor usually displays the deviation value of the ST wave in mm, and this numerical value representing the deviation value of the ST wave is called a ST value. However, in the monitoring process, the ST monitoring function is usually not turned on. Therefore, even if the ST value exceeds the alarm threshold, there still is no alarm that will be triggered. However, sudden myocardial ischemia may cause changes in the ST value. If the ST monitoring function can just be turned on when the user finds the changes in the ST values, the myocardial ischemia of the monitored object may not be detected in time. In this application, if it is found that the deviation value of the ST value satisfies a preset first requirement for an abrupt change during the ECG monitoring, a first indication information which instructs to turn on the ST monitoring function will be given in time, or the ST monitoring function will be turned on directly and intelligently, so as to facilitate the medical staff to detect and analyze a trend of change of the ST value in time. In such a way, when the ST value reaches or exceeds the alarm threshold, an alarm can be generated in time.

Further, when instructing to turn on or intelligently turning on the ST monitoring function, a second indication information is sent at the same time. For example, when the ST monitoring function has been turned on, the deviation of the ST value satisfies a first abrupt change requirement, etc., the second indication information is operable to indicate the user that the data analysis function turned on is the ST monitoring function, the physiological parameter corresponding to the currently turned on data analysis function is the ST value, and the reason for turning on the data analysis function is that the deviation of the ST value satisfies the first abrupt change requirement and so on. In such a way, the patient can be monitored more timely and effectively.

Regarding to the implementation manner of turning on a data measurement function and a data processing function for the first physiological parameter, the following takes the ECG physiological parameter in the ECG signal as the first physiological parameter for example. In a specific example, when the first physiological parameter of the monitored object is an ECG physiological parameter, the real-time analysis function of the ECG physiological parameter is generally turned on. In the real-time analysis and monitoring process of the ECG physiological parameter, it is usually necessary to implement a resting function analysis on the ECG physiological parameter according to the changes of physiological signal characteristics. The resting function analysis is another data function analysis of the ECG physiological parameter, which is different from the real-time ECG function analysis. The resting function analysis is an ECG diagnosis function, which takes the ECG physiological parameters in a certain period of time for analysis and provides the diagnostic result. However, if the set ECG characteristics are not found to change in time, the opportunity to implement the resting function analysis will be missed, and the state changes of the monitored object cannot be tracked in time. To solve this problem, in the embodiment of this disclosure, when the physiological signal characteristics are determined to change according to the analysis of ECG physiological parameter, the resting function analysis will be intelligently instructed to turned on or turned on to implement the data measurement and resting function analysis on the ECG physiological parameter within a preset time period, so as to help the medical staff to track the changes of patient's condition in time and give treatment countermeasures in time.

It can be understood that the first data analysis function or the second data analysis function can also be any combination of at least one of the data measurement function, the data processing function and the monitoring function, and is not limited to the above embodiment.

Regarding to the implementation manner of this step, for example, the at least one monitored parameter includes the first physiological parameter of the monitored object, and the at least one physiological parameter includes the second physiological parameter of the monitored object. After acquiring at least one monitored parameter of the monitored object in the above implementation manner one, when determining that at least one preset physiological signal characteristic changes according to the first physiological parameter, the second data analysis function for the second physiological parameter of the monitored object can be turned on, or the first data analysis function for at least some of the first physiological parameters of the monitored object can be turned off.

In the embodiment of this disclosure, it is possible to determine whether at least one preset physiological signal characteristic changes through analyzing the above at least one monitored parameter.

Here, the preset physiological signal characteristic may include at least one of the followings: a value, a signal waveform shape, and a signal characteristic associated with other physiological parameters, of the at least one monitored parameter. For example, the monitored parameter may be a heart rate, heart rhythm, etc. The value of the at least one monitored parameter includes the value of the physiological parameter and /or the value of the motion state data.

Accordingly, at least one preset physiological signal characteristic changes, includes at least one of the followings:
the value of the at least one monitored parameter changes in accordance with a preset first requirement for an abrupt change;
the signal waveform shape of the at least one monitored parameter changes in accordance with a preset second requirement for an abrupt change; and
the signal characteristic of the at least one monitored parameter which is associated with at least one physiological parameter, changes.

For example, at least one preset physiological signal characteristic changes, includes at least one of the followings:
a heartbeat frequency changes in accordance with a preset first requirement for an abrupt change (the heartbeat frequency changes abruptly);
a signal waveform shape of the first physiological parameter changes in accordance with a preset second requirement for an abrupt change (the signal waveform shape changes abruptly);
a sudden arrhythmia event occurs, and
a signal characteristic of the first physiological parameter which is associated with other physiological parameter, changes.

In an embodiment of this disclosure, the at least one monitored parameter can also be a first physiological parameter of the monitored object, while the at least one physiological parameter includes a second physiological parameter of the monitored object. Accordingly, the first physiological parameter can be analyzed according to a first data analysis function, and when it is determined that at least one preset physiological signal characteristic changes, a second data analysis function for the second physiological parameter of the monitored object can be turned on, or the first data analysis function for at least some of the first physiological parameters of the monitored object can be turned off.

Regarding to the implementation manner of turning on the monitoring function of the second physiological parameter of the monitored object, in one example, a second data analysis function for the second physiological parameter of the monitored object can be turned on according to the physiological signal characteristic which is changed. In practice, the second data analysis function for the second physiological parameter can be intelligently instructed to turn on or turned on. In an embodiment of this disclosure, turning on a data measurement function of the second physiological parameter is taken as an example. When a signal characteristic of the ECG physiological parameter changes, a data measurement function of other physiological parameters (such as NIBP measurement) can be intelligently instructed to turn on or turned on to capture the changes of other physiological parameters when the condition of the monitored object changes. For example, the automatic non-invasive blood pressure (NIBP) measurement can be intelligently instructed to turn on or turned on to capture the changes of the blood pressure of the monitored object in time. It can be understood that the data processing function and/or monitoring function can also be turned on accordingly to process and/or monitor the measured respiratory parameters or blood pressure parameters.

Regarding the implementation manner of turning off the monitoring function of at least some of the first physiological parameters of the monitored object, for example, the monitoring function of at least some of the first physiological parameters of the monitored object can be turned off according to the physiological signal characteristic which is changed. That is, when determining that at least some of the first physiological parameters of the monitored object do not need to be monitored according to the physiological signal characteristic which is changed, the monitoring function of the at least some of the first physiological parameters of the monitored object can be intelligently turned off to reduce the impact of the monitoring function on the monitored object. For example, when it is determined that the condition of the monitored object is improved according to the physiological signal characteristic which is changed, the data analysis function for the at least some of the first physiological parameters can be turned off to reduce the impact of the analysis and measurement on the monitored object and reduce the energy consumption of the device.

Regarding to the implementation manner of turning on the monitoring function of the second physiological parameter of the monitored object, in a further example, a second data analysis function for the second physiological parameter of the monitored object can be turned on, when determining that at least one preset physiological signal feature changes according to an analysis result of the first physiological parameter through the first data analysis function and determining that a change of a second physiological parameter of the monitored object satisfies a preset requirement for a deterioration change according to at least one preset physiological signal characteristic and meanwhile the second data analysis function for the second physiological parameter of the monitored object is turned off.

It can be understood that the change of the second physiological parameter of the monitored object may indicate that the condition of the monitored object is improved or deteriorated. In practice, the preset requirement for the deterioration change can be set in advance according to the monitored parameter of the monitored object. In this way, when the change of the second physiological parameter of the monitored object satisfies the preset requirement for the deterioration change, it indicates that the condition of the monitored object is deteriorating. At this time, it is necessary to turn on the monitoring function of the second physiological parameter of the monitored object. Furthermore, when the monitoring function of the second physiological parameter of the monitored object is turned off, the monitoring function of the second physiological parameter of the monitored object is turned on intelligently, so as to timely capture the causes of the deterioration of the condition of the monitored object. For example, for the second physiological parameter of the monitored object, when no alarm is generated due to the turning off of the alarm switch, the corresponding alarm switch can be intelligently instructed to turn on or directly turned on for turning the corresponding alarm function.

After turning on the second data analysis function for at least one physiological parameter of the monitored object, the method also includes generating an alarm information when a monitored physiology satisfies a preset alarm requirement. In the embodiment of this disclosure, the implementation manner of the alarm function is not limited.

The implementation manner for turning on a monitoring function of the second physiological parameter of the monitored object is described by a specific example below.

During a routine ECG monitoring process, only the heart rate and arrhythmia are usually monitored. In many application scenarios, the arrhythmia switch adopts a default setting, that is, only pay attention to whether the monitored object has a fatal arrhythmia (such as asystolia, ventricular fibrillation, ventricular tachycardia, extreme tachycardia or bradycardia), but for non-fatal arrhythmia (non-persistent ventricular tachycardia, multi-geminy, bigeminy, trigeminy, atrial fibrillation, irregular rhythm, etc.), the monitoring function is basically turned off. However, when the occurrence of some arrhythmias (such as bigeminy) causes a hemodynamic change of the monitored object, at this time, if the monitoring function of the bigeminy is still turned off, a risky alarm may be omitted, which is not conducive to the medical staff to find out what causes the deterioration of the monitored object in time. With the technical scheme of the embodiment of this disclosure, due to the real-time monitoring of the ECG physiological parameter and hemodynamic characteristic, when the hemodynamic characteristic changes, the monitoring function of the bigeminy can intelligently be instructed to turn on or turned on, so that the alarm event of the bigeminy can be captured in time. In such a way, a timely reminder can be sent to the medical staff. Further, a second indication information can be sent to instruct the user the monitoring function, monitored physiological parameter and so on. For example, the second indication information can instruct the user that the reason for turning on the monitoring function of the bigeminy is the change in the hemodynamic characteristics.

Further, the alarm function for the physiological parameter can be realized when the monitored physiological parameter satisfies a preset alarm requirement. In the embodiment of this disclosure, the implementation manner of the alarm function is not limited.

The embodiment of this disclosure is not limited to the ECG monitoring scene, such as the ECG monitoring in the CCU scenes. In the other scenes, the blood oxygen of the monitored object may not be necessarily monitored when using a ventilator to assist breathing, or the blood oxygen probe may be removed due to the uncomfortable wearing of the monitored object. The oxygen supply of the monitored object may be affected when the monitored object has trespiratory asphyxia. At this time, it is necessary to instruct the medical staff to monitor the blood oxygen of the monitored object in time to check the oxygen supply state of the monitored object. Now, the data analysis function for the blood oxygen can be turned on intelligently, or the data analysis function for the blood oxygen can be turned on by sending the first indication information. Here, the data analysis function can include the data measurement function, data processing and monitoring function at the same time. When the data analysis function for the blood oxygen is turned on, the alarm rules of the blood oxygen monitoring are implemented. For example, if the blood oxygen probe is normally worn, the blood oxygen monitoring is carried out. When the blood oxygen reaches an alarm threshold and the preset alarm requirement is satisfied, then an alarm information is generated to instruct that the blood oxygen reaches or exceeds the threshold. If the blood oxygen probe is not normally worn, the blood oxygen value is invalid or there is no signal. At this time, it also satisfies the preset relevant alarm requirement, then the alarm information is also generated to instruct that the blood oxygen probe is not normally worn.

In conclusion, by adopting the technical scheme of the embodiment of this disclosure, when a certain monitored parameter of the monitored object changes, the medical staff can be can adaptively instructed to monitor other physiological parameters in time, or the data analysis function for some physiological parameter associated with the monitored parameter can be intelligently turned on, so as to timely capture the change of the state of the monitored object based on the indication or performance of more parameters, thus improving the diagnosis and treatment efficiency of the disease, and making the monitor more intelligent and more in line with the operation habits of the medical staff.

Further, when at least one preset physiological signal characteristic is determined to be stable according to the above at least one monitored parameter, the first data analysis function for at least some of the first physiological parameters of the monitored object can be turned off. It can be seen that, when at least one preset physiological signal characteristic is stable (for example, the signal waveform shape of at least one monitored parameter is always in a stable state), the state of the monitored object can be determined as being basically stable. At this time, by turning off the first data analysis function for at least some of the first physiological parameters of the monitored object, the impact of the monitoring or alarm function on the monitored object can be reduced.

For example, in the above example, if the heart rate/pulse rate parameter characteristics are basically stable and basically consistent with the previous NIBP measurement, then the NIBP measurement function can be intelligently turned off to reduce the impact on the patient during NIBP measurement. Similarly, when the resting function analysis is turned on and the monitored physiological signal characteristic of the ECG physiological parameter tends to be or is already in a normal or stable state, the resting function analysis can be intelligently turned off to reduce the impact of the analysis and measurement on the patient and to reduce the energy consumption of the device.

Regarding the implementation manner of step 102, for example, after acquiring the motion state data and/or posture state data of the monitored object in the above implementation manner two, the second data analysis function for at least one physiological parameter of the monitored object can be turned on or off according to the motion state data and/or posture state data of the monitored object. That is, by adopting the technical scheme of the embodiment of this disclosure, the second data analysis function for the physiological parameter can be intelligently turned on or off in combination with the motion state (stable or violent) and/or posture information of the monitored object. For example, when the monitored object is often in strenuous exercise, the measurement function of the physiological parameter of the monitored object can be turned off, which can prevent the measurement result of the physiological parameter of the monitored object from affecting the determination of the medical staff when the monitored object is in frequent exercise.

The first physiological parameter and the second physiological parameter include at least one of the followings: an ECG physiological parameter, a respiratory parameter, a blood pressure, a blood oxygen, a pulse, a body temperature, end expiratory carbon dioxide, an anesthetic gas, a cardiac output, a bispectral index of EEG, etc.

In practical application, steps 101 and 102 can be implemented by a device, such as a processor in the monitor. The above processor can be at least one of: an application specific integrated circuit (ASIC), digital signal processor (DSP), digital signal processing device (DSPD) and programmable logic device (PLD), field programmable gate array (FPGA), central processing unit (CPU), controller, microcontroller, microprocessor, etc.

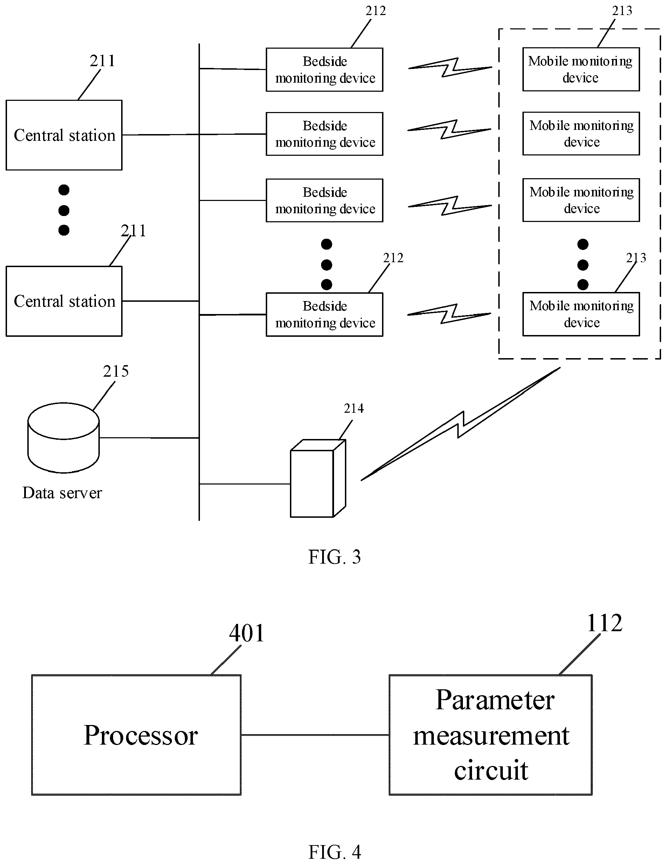

The application scenario of the embodiment of this disclosure is described below through FIG. 2 and FIG. 3.

FIG. 2 is a system framework diagram of a multi-parameter monitor or module component according to an embodiment of this disclosure. As shown in FIG.2, the multi-parameter monitor or module component at least includes a parameter measurement circuit 112.

The parameter measurement circuit 112 at least includes a parameter measurement circuit corresponding to at least one parameter. The parameter measurement circuit at least includes an ECG parameter measurement circuit, a respiratory parameter measurement circuit, a body temperature parameter measurement circuit, a blood oxygen parameter measurement circuit, a noninvasive blood pressure parameter measurement circuit, an invasive blood pressure parameter measurement circuit, a motion signal measurement circuit and so on. Each parameter measurement circuit is respectively connected with a sensor accessory 111 externally inserted through a corresponding sensor interface. The sensor accessory 111 includes at least one of detection accessories corresponding to the detection of the physiological parameters such as ECG, respiration, blood oxygen, blood pressure and body temperature. The parameter measurement circuit 112 is mainly operable to connect the sensor accessory 111 to acquire acquired physiological parameter signal and may include a parameter measurement circuit corresponding to at least one parameter. The parameter measurement circuit 112 may be, but is not limited to, a physiological parameter measurement circuit (module), a human physiological parameter measurement circuit (module) or a sensor for acquiring human physiological parameter or non-physiological parameter. Specifically, the parameter measurement circuit obtains a physiological sampling signal related to a patient from an external physiological parameter sensor accessory by means of an extended interface, and then obtains physiological data after processing the physiological sampling signal for alarming and displaying. The extended interface can also be operable to output a control signal which is about how to acquire the physiological parameter and is outputted by a main control circuit to an external physiological parameter monitoring accessory through a corresponding interface, so as to control the monitoring of the physiological parameters of the patient.

The multi-parameter monitor or module component may include a main control circuit 113, which needs to include at least one processor and at least one memory. Of course, the main control circuit 113 may also include at least one of a power management module, a power IP module, an interface conversion circuit, etc. The power management module is operable to control the power-on and power-off operation of the whole machine, a power-on time sequence of each power domain inside a board card and battery charging and discharging, etc. The power IP module is a separate power module firmed by associating a principle diagram of a power supply circuit unit that is frequently and repeatedly invoked, with a PCB layout. That is, an input voltage is converted into an output voltage by means of a predetermined circuit, where the input voltage and the output voltage are different. For example, the voltage of 15 V is converted into 1.8 V, 3.3 V, 3.8 V, etc. It can be understood that the power IP module may be single-channel or multi-channel. If the power IP module is single-channel, the power IP module may convert one input voltage into one output voltage. If the power IP module is multi-channel, the power IP module may convert one input voltage into a plurality of output voltages, and voltage values of the plurality of output voltages may be the same or different, such that different voltage requirements of a plurality of electronic components can be met at the same time. In addition, the module has few external interfaces, and works in the system as a black box decoupled from an external hardware system, which improves the reliability of the entire power system. The interface conversion circuit is operable to convert a signal output by a master control minimum system module (i.e., at least one processor and at least one memory in the main control circuit 113) into an input standard signal required to be received by an actual external device. For example, supporting an external VGA display function is to convert an RGB digital signal output by a master control CPU into a VGA analog signal, and supporting an external network function is to convert an RMII signal into a standard network differential signal.

In addition, the multi-parameter monitor or module component may also include one or more of a local display 114, an alarm circuit 116, an input interface circuit 117, and an external communication and power interface 115. The main control circuit 113 is operable to coordinate and control each board card, circuit and device in a multi-parameter monitoring apparatus or the module assembly. In this embodiment, the main control circuit 113 is operable to control data interaction and control signal transmission between the parameter measurement circuit 112 and a communication interface circuit, and transmit the physiological data to the display 114 for display, or may receive a user control instruction entered from a touchscreen or a physical input interface such as a keyboard and keys, and may also output a control signal for acquiring the physiological parameters. The alarm circuit 116 may be an audible and visual alarm circuit. The main control circuit 113 completes the calculation of the physiological parameters, and may send calculation results and waveforms of the parameters to the host (such as a host with a display, a PC, and a central station and so on) by means of the external communication and power interface 115. The external communication and power interface 115 may be one or a combination of local area network interfaces composed of Ethernet, a token ring, a token bus, and an optical fiber distributed data interface (FDDI) as the backbone of these three networks, or may be one or a combination of wireless interfaces such as infrared, Bluetooth, WIFI, and WMTS communication, or may be one or a combination of wired data connection interfaces such as RS232 and USB. The external communication and power interface 115 may also be one of a wireless data transmission interface and a wired data transmission interface or a combination thereof. The host may be any computing device, such as a host of the monitoring apparatus, an electrocardiograph machine, an ultrasonic diagnosis instrument, and a computer, and can form a monitoring apparatus once installed with matching software. The host may alternatively be a communication device such as a mobile phone, and the multi-parameter monitoring apparatus or the module assembly sends, by means of a Bluetooth interface, data to the mobile phone supporting Bluetooth communication, so as to implement remote transmission of data.

The multi-parameter monitor component may be arranged outside a housing of the monitor as an independent externally inserted parameter module, may be inserted into the host (including a main control board) of the monitor to form a plug-in monitor as a part of the monitor, or may be connected to the host (including the main control board) of the monitor by means of a cable, and the externally inserted parameter module is used as an external accessory of the monitor. Alternatively, the parameter processing module may also be built in the housing to be integrated with a main control module, or physically separated and arranged in the housing to form an integrated monitor.

As shown in FIG. 3, a monitor networking system for use in a hospital is provided. By using the monitor networking system, the data of a monitor may be saved as a whole, where patient information and nursing information can be managed centrally and may be stored in association, which facilitates the storage of historical data and associated alarming. In the system shown in FIG. 3, a bedside monitor 212 may be provided for each hospital bed. The bedside monitor 212 can be any one of the above multi-parameter monitor or plug-in monitor. In addition, each bedside monitor 212 may further be paired with a mobile monitor 213 for transmission. The mobile monitor 213 provides a simple and portable multi-parameter monitor or module component and can be worn on the body of a patient for mobile monitoring of the patient. With the wired or wireless communication between the mobile monitor 213 and the bedside monitor 212, physiological data generated during the mobile monitoring process may be transmitted to the bedside monitor 212 for displaying, or transmitted through the bedside monitor 212 to a central station 211 for a doctor or nurse to check, or transmitted through the bedside monitor 212 to a data server 215 for storage. In addition, the mobile monitor 213 may further directly transmit, through a wireless network node 214 arranged in the hospital, the physiological data generated during the mobile monitoring process to the central station 211 for storage and display, or transmit, through the wireless network node 214 arranged in the hospital, the physiological data generated during the mobile monitoring process to the data server 215 for storage. It can be seen that the data corresponding to the physiological parameter displayed on the bedside monitor 212 may originate from the sensor accessory directly connected to the monitor, or from the mobile monitor 213, or from the data server.

It should be noted that the application scenarios shown in FIGS. 2 and 3 are only an exemplary application scenario of the embodiment of this disclosure, and the embodiment of this disclosure is not limited to this. Based on the application scenarios shown in FIGS. 2 and 3 and the method for operating a monitoring apparatus proposed in the above embodiment, the embodiment of this disclosure further proposes a monitoring apparatus as follows.

Fig. 4 is a structural diagram of a monitoring apparatus according to an embodiment of this disclosure. As shown in FIG. 4, the monitoring apparatus may include a processor 401 and a parameter measurement circuit 112.

Among them, the parameter measurement circuit 112 is configured to acquire at least one monitored signal of a monitored object in real time through a sensor accessory connected with the monitored object in a monitoring process of the monitored object.

The processor 401 is configured to acquire at least one monitored parameter of the monitored object according to the monitored signal, and analyze the at least one monitored parameter according to a first data analysis function; wherein when the at least one monitored parameter satisfies a preset requirement, the processor is configured to perform a data analysis function change step, wherein the data analysis function change step includes at least one of the following steps:
turning on a second data analysis function for at least one physiological parameter for indicating a real-time change of the at least one physiological parameter; and
turning off the second data analysis function for at least one physiological parameter;
wherein the monitored parameter is at least operable to characterize at least one of following characteristics of the monitored object: a physiological state, a motion state and a posture state.

In one embodiment, the first data analysis function and the second data analysis function include at least one of the following functions: a data measurement function for acquiring a physiological characteristic signal in real time to acquire at least one physiological parameter according to the physiological characteristic signal; a data processing function for processing at least one physiological parameter according to a preset rule; and a monitoring function for generating an alarm information when at least one physiological parameter satisfies a preset alarm requirement.

In one embodiment, the at least one monitored parameter includes a first physiological parameter for characterizing a physiological state of the monitored object, the at least one physiological parameter includes the first physiological parameter.

Correspondingly, the processor 401 is specifically configured to turn on or off the second data analysis function for the first physiological parameter when analyzing a change of the first physiological parameter according to the first data analysis function and determining that at least one preset physiological signal characteristic changes.

In one embodiment, the at least one monitored parameter is the first physiological parameter of the monitored object, and the at least one physiological parameter includes a second physiological parameter of the monitored object.

Correspondingly, the processor 401 is specifically configured to turn on the second data analysis function for the second physiological parameter of the monitored object or turn off the first data analysis function for at least some of the first physiological parameters of the monitored object, when analyzing the first physiological parameter according to the first data analysis function and determining that at least one preset physiological signal characteristic changes.

In one embodiment, the at least one monitored parameter is the first physiological parameter of the monitored object, and the at least one physiological parameter includes the second physiological parameter of the monitored object.

Correspondingly, the processor 401 is specifically configured to analyze the first physiological parameter according to the first data analysis function, determine that at least one preset physiological signal characteristic changes, and turn on the second data analysis function for the second physiological parameter of the monitored object, when determining that a change of a second physiological parameter of the monitored object satisfies a preset requirement for a deterioration change according to the change of the at least one preset physiological signal characteristic and meanwhile the second data analysis function for the second physiological parameter of the monitored object is turned off.

In an embodiment, at least one preset physiological signal characteristic changes, includes at least one of the followings:
a value of the at least one monitored parameter changes in accordance with a preset first requirement for an abrupt change; a signal waveform shape of the at least one monitored parameter changes in accordance with a preset second requirement for an abrupt change; and a signal characteristic of the at least one monitored parameter which is associated with at least one physiological parameter, changes.

In one embodiment, the processor 401 is further configured to turn off the first data analysis function for at least some of the first physiological parameters of the monitored object when analyzing the at least one monitored parameter according to the first data analysis function, and determining that at least one preset physiological signal characteristic is stable.

In one embodiment, the first physiological parameter is determined according to an actual monitoring requirement of the monitored object.

In one embodiment, the processor 401 is further configured to send a second indication information when the second data analysis function for at least one physiological parameter of the monitored object is turned on or off. Wherein, the second indication information includes at least one of the followings: the preset requirement, the monitored parameter, the physiological parameter, and the data analysis function which is turned on or off.

In one embodiment, the form of the second indication information includes at least one of the following: sound, light, text and graphics.

In one embodiment, the parameter measurement circuit 112 includes a motion sensor, and the monitored signal includes at least one motion signal. Accordingly, the processor is specifically configured to acquire a motion state data and/or a posture state data of the monitored object according to the at least one motion signal, analyze the motion state data and/or the posture state data of the monitored object according to the first data analysis function, and turn on or off the second data analysis function for at least one physiological parameter of the monitored object.

In one embodiment, the processor 401 is specifically configured to:
send a first indication information which instructs to turn on or off a second data analysis function for at least one physiological parameter of the monitored object when the at least one monitored parameter satisfies a preset requirement; and
turn on or off a data analysis function for at least one physiological parameter of the monitored object after receiving a confirmation instruction.

In one embodiment, the form of the first indication information includes at least one of the following: sound, light, text and graphics.

In one embodiment, the processor 401 is further configured to generate an alarm information after turning on the second data analysis function for at least one physiological parameter of the monitored object and when the monitored physiological parameter satisfies a preset alarm requirement.

In practical application, the processor 401 can be at least one of ASIC, DSP, DSPD, PLD, FPGA, CPU, controller, microcontroller, microprocessor, etc.

In essence, the technical solution of this embodiment or the part of this embodiment that contributes to the prior art, or all or part of the technical solution can be embodied in the form of a computer software product. The computer software product can be stored in a storage medium, including several instructions, to enable a computer device (which can be a personal computer, a server, a network device, etc.) or a processor to perform all or some of the steps of the method described in the present embodiment. The aforementioned storage media include: a USB flash disk, mobile hard disk, read only memory (ROM), random access memory (RAM), magnetic disc or optical disc and other media that can store program codes.

Specifically, the computer program instructions corresponding to the method for operating a monitoring apparatus in this embodiment can be stored on a storage media, such as an optical disc, hard disk and USB flash disk and so on. When the computer program instructions corresponding to the method for operating a monitoring apparatus in the storage media are read or executed by an electronic device, the method for operating a monitoring apparatus according to any one of the above embodiments can be realized.

The embodiment further provides a monitor, including anyone monitoring apparatus recorded in the above embodiments.

The methods disclosed in the method embodiments provided in the present application can be combined arbitrarily to acquire a new method embodiment, without conflicting to each other.

The features disclosed in the product embodiments provided by the application can be combined arbitrarily obtain a new product embodiment, without conflicting to each other.

The features disclosed in the methods or product embodiments provided in the application can be combined arbitrarily obtain a new method embodiment or equipment embodiment, without conflicting to each other.

In several embodiments provided by this disclosure, it should be understood that the disclosed method and intelligent device can be realized in other ways. The device embodiments described above are only schematic. For example, the division of the units is only a logical function division, and there can be another division mode in the actual implementation, such as multiple units or components can be combined or integrated into another system, or some features can be ignored or not executed. In addition, the coupling, direct coupling or communication connection between the components shown or discussed can be through some interfaces, indirect coupling or communication connection of equipment or units, and can be electrical, mechanical or other forms.

The units described above as separate components may or may not be physically separated, and the components displayed as units may or may not be physical units, that is, they may be located in one place or distributed over multiple network units. Some or all of the units can be selected according to actual needs to achieve the purpose of the embodiment.

In addition, in each embodiment of this disclosure, all functional units can be integrated in a second processing unit, each unit can be used as a unit separately, or two or more units can be integrated in one unit. The above integrated units can be realized in the form of a hardware or a hardware plus a software functional unit.

The above is only the specific embodiment of this disclosure, but the protection scope of this disclosure is not limited to this. Any person of ordinary skill in the art could also make several variations and improvements without departing from the concept of this disclosure, and these variations and improvements would all fall within the scope of protection of this disclosure.

## Claims

1. A monitoring apparatus comprising a processor and a parameter measurement circuit; wherein
the parameter measurement circuit is configured to acquire a monitored signal of a monitored object in real time through a sensor accessory connected with the monitored object in a monitoring process of the monitored object;
the processor is configured to acquire a monitored parameter of the monitored object according to the monitored signal, and analyze the monitored parameter according to a first data analysis function; wherein the monitored parameter includes at least a first physiological parameter which characterizes a physiological state of the monitored object;
wherein when the monitored parameter satisfies a preset requirement, the processor is further configured to perform a data analysis function change step, wherein the data analysis function change step includes at least one of following steps:
turning on a second data analysis function for the first physiological parameter; and
turning off the second data analysis function for the first physiological parameter.

2. The monitoring apparatus according to claim 1, wherein the first data analysis function and the second data analysis function include at least one of following functions:
a data measurement function which acquires a physiological characteristic signal in real time to acquire the first physiological parameter according to the physiological characteristic signal;
a data processing function which processes the first physiological parameter according to a preset rule; and
a monitoring function which generates an alarm information when the first physiological parameter satisfies a preset alarm requirement.

3. The monitoring apparatus according to claim 1, wherein the processor is specifically configured to turn on or off the second data analysis function for the first physiological parameter, when analyzing that the first physiological parameter changes according to the first data analysis function and determining that at least one preset physiological signal characteristic changes.

4. The monitoring apparatus according to claim 3, wherein that at least one preset physiological signal characteristic changes, comprises at least one of:
a value of the first physiological parameter changes in accordance with a first preset requirement for an abrupt change;
a signal waveform shape of the first physiological parameter changes in accordance with a preset second requirement for an abrupt change; and
a signal characteristic of the first physiological parameter, which is associated with a second physiological parameter, changes.

5. The monitoring apparatus according to claim 3, wherein the processor is further configured to turn off the second data analysis function for at least some of the first physiological parameters, when analyzing the first physiological parameter according to the first data analysis function and determining that at least one set physiological signal characteristic is stable.

6. The monitoring apparatus according to claim 1, wherein the processor is further configured to send a second indication information when the second data analysis function for the first physiological parameter is turned on or off;
wherein, the second indication information comprises at least one of: the preset requirement, the monitored parameter, the first physiological parameter, and the second data analysis function which is turned on or off.

7. The monitoring apparatus according to claim 1, wherein the processor is specifically configured to :
send a first indication information which instructs to turn on or off the second data analysis function for the first physiological parameter when determining that the first physiological parameter satisfies the preset requirement according to the first data analysis function; and
turn on or off the second data analysis function for the first physiological parameter after receiving a confirmation instruction.

8. A method for operating a monitoring apparatus, comprising:
acquiring at least one monitored signal of a monitored object in real time in a monitoring process of the monitored object;
acquiring a monitored parameter of the monitored object according to the monitored signal, and analyzing the monitored parameter according to a first data analysis function; wherein the monitored parameter includes at least a first physiological parameter which characterizes a physiological state of the monitored object;
wherein when the monitored parameter satisfies a preset requirement, performing a data analysis function change step, wherein the data analysis function change step includes at least one of following steps:
turning on a second data analysis function for the first physiological parameter;
turning off the second data analysis function for the first physiological parameter.

9. The method according to claim 8, wherein the first data analysis function and the second data analysis function include at least one of following functions:
a data measurement function which acquires a physiological characteristic signal in real time to acquire at least one physiological parameter according to the physiological characteristic signal;
a data processing function which processes at least one physiological parameter according to a preset rule; and
a monitoring function which generates an alarm information when at least one physiological parameter satisfies a preset alarm requirement.

10. The method according to claim 8, wherein when the monitored parameter satisfies a preset requirement, performing a data analysis function change step, comprises:
turning on or off the second data analysis function for the first physiological parameter, when analyzing that the first physiological parameter changes according to the first data analysis function and determining that at least one preset physiological signal characteristic changes.

11. The method according to claim 10, wherein at least one preset physiological signal characteristic changes, comprises at least one of:
a value of the first physiological parameter changes in accordance with a preset first requirement for an abrupt change;
a signal waveform shape of the first physiological parameter changes in accordance with a preset second requirement for an abrupt change; and
a signal characteristic of the first physiological parameter which is associated with a second physiological parameter changes.

12. The method according to claim 10, wherein when the monitored parameter satisfies a preset requirement, performing a data analysis function change step further comprises:
turning off the second data analysis function for at least some of the first physiological parameters, when analyzing the monitored parameter according to the first data analysis function and determining that at least one preset physiological signal characteristic is stable.

13. The method according to claim 8, further comprising:
sending a second indication information when turning on or off the second data analysis function for the first physiological parameter; wherein, the second indication information comprises at least one of: the preset requirement, the monitored parameter, the first physiological parameter, and the second data analysis function which is turned on or off.

14. The method according to claim 8, wherein acquiring at least one monitored parameter of the monitored object, further comprises: acquiring a motion state data and/or a posture state data of the monitored object;
correspondingly, performing a data analysis function change step according to the at least one monitored parameter, comprises:
analyzing the motion state data and/or the posture state data of the monitored object according to the first data analysis function; and
turning on or off a data analysis function for at least one physiological parameter of the monitored object.

15. The method according to claim 8, wherein when determining that at least one monitored parameter satisfies the preset requirement according to the first data analysis function, performing a data analysis function change step, comprises:
sending a first indication information which instructs to turn on or off the second data analysis function for the first physiological parameter of the monitored object when the at least one monitored parameter satisfies the preset requirement; and
turning on or off the second data analysis function for the first physiological parameter after receiving a confirmation instruction.

16. A monitoring apparatus comprising a processor and a parameter measurement circuit; wherein
the parameter measurement circuit is configured to acquire at least one monitored signal of a monitored object in real time through a sensor accessory connected with the monitored object in a monitoring process of the monitored object;
the processor is configured to acquire at least one monitored parameter of the monitored object according to the monitored signal, and analyze the at least one monitored parameter according to a first data analysis function; wherein when the at least one monitored parameter satisfies a preset requirement, the processor is configured to perform a data analysis function change step wherein the data analysis function change step includes at least one of following steps:
turning on a second data analysis function for at least one physiological parameter of the monitored object; and
turning off the second data analysis function for at least one physiological parameter of the monitored object;
wherein the monitored parameter is at least operable to characterize at least one of following characteristics of the monitored object: a physiological state, a motion state and a posture state.

17. The monitoring apparatus according to claim 16, wherein the first data analysis function and the second data analysis function include at least one of following functions:
a data measurement function which acquires a physiological characteristic signal in real time to acquire the at least one physiological parameter according to the physiological characteristic signal;
a data processing function which processes the at least one physiological parameter according to a preset rule; and
a monitoring function which generates an alarm information when the at least one physiological parameter satisfies a preset alarm requirement.

18. The monitoring apparatus according to claim 16, wherein the at least one monitored parameter includes a first physiological parameter which characterizes a physiological state of the monitored object; and the at least one physiological parameter includes the first physiological parameter;
correspondingly, the processor is specifically configured to turn on or off the second data analysis function for the first physiological parameter, when analyzing that the first physiological parameter changes according to the first data analysis function and determining that at least one preset physiological signal characteristic changes.

19. The monitoring apparatus according to claim 16, wherein the at least one physiological parameter is a first physiological parameter of the monitored object; and the at least one physiological parameter includes a second physiological parameter of the monitored object;
correspondingly, the processor is specifically configured to turn on the second data analysis function for the second physiological parameter or turn off the first data analysis function for at least some of the first physiological parameters when analyzing the first physiological parameter according to the first data analysis function and determining that at least one preset physiological signal characteristic changes.

20. The monitoring apparatus according to claim 16, wherein the at least one physiological parameter is a first physiological parameter of the monitored object; and the at least one physiological parameter includes a second physiological parameter of the monitored object;
correspondingly, the processor is specifically configured to turn on the second data analysis function for the second physiological parameter; when analyzing the first physiological parameter according to the first data analysis function, determining that at least one preset physiological signal characteristic changes and further determining that a change of the second physiological parameter of the monitored object satisfies a preset requirement for a deterioration change according to the change of the at least one preset physiological signal characteristic and meanwhile the second data analysis function for the second physiological parameter of the monitored object is turned off.

21. The monitoring apparatus according to any one of claims 17-20, wherein at least one preset physiological signal characteristic changes, comprises at least one of:
a value of the at least one monitored parameter changes in accordance with a preset first requirement for an abrupt change;
a signal waveform shape of the at least one monitored parameter changes in accordance with a preset second requirement for an abrupt change; and
a signal characteristic of the at least one monitored parameter which is associated with at least one physiological parameter, changes.

22. The monitoring apparatus according to any one of claims 17-20, wherein the processor is further configured to turn off the first data analysis function for at least some of the first physiological parameters of the monitored object, when analyzing the at least one physiological parameter according to the first data analysis function and determining that at least one preset physiological signal characteristic is stable.

23. The monitoring apparatus according to any one of claims 17-20, wherein the first physiological parameter is determined according to an actual requirement of the monitored object.

24. The monitoring apparatus according to claim 16, wherein the processor is further configured to send a second indication information when the second data analysis function for the at least one physiological parameter of the monitored object is turned on or off; wherein, the second indication information comprises at least one of: the preset requirement, the monitored parameter, the physiological parameter, and the second data analysis function which is turned on or off.

25. The monitoring apparatus according to claim 24, wherein the second indication information is in a form of: sound, light, text or graphics.

26. The monitoring apparatus according to claim 16, wherein the parameter measurement circuit comprises a motion sensor and the monitored signal includes at least one motion signal;
correspondingly, the processor is specifically configured to acquire a motion state data and/or a posture state data of the monitored object according to the at least one motion signal, analyze the motion state data and/or the posture state data of the monitored object according to the first data analysis function, and turn on or off the second data analysis function for the at least one physiological parameter of the monitored object.

27. The monitoring apparatus according to claim 16, wherein the processor is specifically configured to :
send a first indication information which instructs to turn on or off the second data analysis function for the at least one physiological parameter of the monitored object when determining that the at least one monitored parameter satisfies the preset requirement; and
turn on or off a data analysis function for the at least one physiological parameter of the monitored object after receiving a confirmation instruction.

28. The monitoring apparatus according to claim 17, wherein the second indication information is in a form of: sound, light, text or graphics.

29. The monitoring apparatus according to claim 16, wherein the processor is further configured to generate an alarm information after turning on the second data analysis function for the at least one physiological parameter of the monitored object and meanwhile monitored physiological parameter satisfies a preset alarm requirement.

30. A monitor comprising the monitoring apparatus according to any one of claims 1-7 or claims 26-29.

31. A method for operating a monitoring apparatus, comprising:
acquiring at least one monitored signal of a monitored object in real time through a sensor accessory connected with the monitored object in a monitoring process of the monitored object;
acquiring at least one monitored parameter of the monitored object according to the monitored signal, and analyzing the at least one monitored parameter according to a first data analysis function; wherein when the at least one monitored parameter satisfies a preset requirement, performing a data analysis function change step, wherein the data analysis function change step includes at least one of following steps:
turning on a second data analysis function for at least one physiological parameter of the monitored object; and
turning off the second data analysis function for at least one physiological parameter of the monitored object;
wherein the monitored parameter is at least operable to characterize at least one of following characteristics of the monitored object: a physiological state, a motion state and a posture state.

32. The method according to claim 31, wherein the first data analysis function and the second data analysis function include at least one of following functions:
a data measurement function which acquires a physiological characteristic signal in real time to acquire the at least one physiological parameter according to the physiological characteristic signal;
a data processing function which processes the at least one physiological parameter according to a preset rule; and
a monitoring function which generates an alarm information when the at least one physiological parameter satisfies a preset alarm requirement.

33. The method according to claim 31, wherein the at least one monitored parameter includes a first physiological parameter which characterizes a physiological state of the monitored object; and the at least one physiological parameter includes the first physiological parameter;
correspondingly, when the at least one monitored parameter satisfies a preset requirement, performing a data analysis function change step, comprises:
turning on or off the second data analysis function for the first physiological parameter, when analyzing that the first physiological parameter changes according to the first data analysis function and determining that at least one preset physiological signal characteristic changes.

34. The method according to claim 31, wherein the at least one monitored parameter includes a first physiological parameter of the monitored object, and the at least one physiological parameter includes a second physiological parameter of the monitored object;
correspondingly, when the at least one monitored parameter satisfies a preset requirement, performing a data analysis function change step, comprises:
turning on the second data analysis function for the second physiological parameter or turning off the first data analysis function for at least some of the first physiological parameters when analyzing the first physiological parameter according to the first data analysis function and determining that at least one preset physiological signal characteristic changes.

35. The method according to claim 31, wherein the at least one monitored parameter is a first physiological parameter of the monitored object, and the at least one physiological parameter includes a second physiological parameter of the monitored object;
correspondingly, when the at least one monitored parameter satisfies a preset requirement, performing a data analysis function change step, comprises:
turning on the second data analysis function for the second physiological parameter; when analyzing the first physiological parameter according to the first data analysis function, determining that at least one preset physiological signal characteristic changes and further determining that a change of the second physiological parameter of the monitored object satisfies a preset requirement for a deterioration change according to the change of the at least one preset physiological signal characteristic and meanwhile the second data analysis function for the second physiological parameter of the monitored object is turned off.

36. The method according to any one of claims 32-35, wherein at least one preset physiological signal characteristic changes, comprises at least one of:
a value of the at least one monitored parameter changes in accordance with a preset first requirement for an abrupt change;
a signal waveform shape of the at least one monitored parameter changes in accordance with a preset second requirement for an abrupt change; and
a signal characteristic of the at least one monitored parameter which is associated with at least one physiological parameter, changes.

37. The method according to any one of claims 32-35, wherein when the at least one monitored parameter satisfies a preset requirement, performing a data analysis function change step further comprises:
turning off the first data analysis function for at least some of the first physiological parameters, when analyzing the at least one physiological parameter according to the first data analysis function and determining that at least one preset physiological signal characteristic is stable.

38. The method according to any one of claims 32-35, wherein the physiological parameter is determined according to actual requirement of the monitored object.

39. The method according to claim 31, further comprising:
sending a second indication information when the second data analysis function for the at least one physiological parameter of the monitored object is turned on or off; wherein, the second indication information comprises at least one of: the preset requirement, the monitored parameter, the physiological parameter, and the second data analysis function which is turned on or off.

40. The method according to claim 39, wherein the second indication information is in a form of: sound, light, text or graphics.

41. The method according to claim 31, wherein the monitored signal includes at least one motion signal;
acquiring at least one monitored parameter of the monitored object, comprises: acquiring a motion state data and/or a posture state data of the monitored object according to the at least one motion signal;
correspondingly, when the at least one monitored parameter satisfies a preset requirement, performing a data analysis function change step further comprises:
analyzing the motion state data and/or the posture state data of the monitored object according to the first data analysis function and turning on or off the second data analysis function for the at least one physiological parameter of the monitored object.

42. The method according to claim 31, wherein when the at least one monitored parameter satisfies a preset requirement, performing a data analysis function change step further comprises:
sending a first indication information which instructs to turn on or off the second data analysis function for the at least one physiological parameter of the monitored object, when determining that the at least one monitored parameter satisfies the preset requirement; and
turning on or off a data analysis function for the at least one physiological parameter of the monitored object after receiving a confirmation instruction.

43. The method according to claim 42, wherein the second indication information is in a form of: sound, light, text or graphics.

44. The method according to claim 41, further comprising:
generating an alarm information after turning on the second data analysis function for the at least one physiological parameter of the monitored object and meanwhile monitored physiological parameter satisfies a preset alarm requirement.

45. A computer storage medium comprising a program that can be executed by to implement the method according to any one of claims 8-15 or claims 31-44.
